# EUROPÄISCHE PATENTSCHRIFT

(11) **EP 3 282 255 B1**
(45) Veröffentlichungstag und Bekanntmachung des Hinweises auf die Patenterteilung: **09.10.2019**
(21) Anmeldenummer: 16184020.2
(22) Anmeldetag: 12.08.2016
(51) Int. Cl.: G01N 33/543, C12N 11/08

(54) **VERFAHREN ZUR HERSTELLUNG EINES PROTEIN-FUNKTIONALISIERTEN FILMS SOWIE PROTEIN-FUNKTIONALISIERTER FILM**
METHOD FOR PRODUCING A PROTEIN FUNCTIONALISED FILM AND PROTEIN FUNCTIONALISED FILM
PROCEDE DE FABRICATION D'UN FILM FONCTIONNALISE PAR PROTEINE ET FILM FONCTIONNALISE PAR PROTEINE

(43) Veröffentlichungstag der Anmeldung: 14.02.2018
(73) Patentinhaber: Fraunhofer-Gesellschaft zur Förderung der angewandten Forschung e.V., 80686 München (DE)
(72) Erfinder: Reinicke, Stefan, 14473 Potsdam (DE); Böker, Alexander, 13156 Berlin (DE)
(74) Vertreter: Pfenning, Meinig & Partner mbB

(56) Entgegenhaltungen:
- US-A- 3 171 831
- HAOYU WANG ET AL: "Elastic Properties of Protein Functionalized Nanoporous Polymer Films", LANGMUIR, Bd. 32, Nr. 1, 12. Januar 2016 (2016-01-12), Seiten 151-158, XP055320359, US ISSN: 0743-7463, DOI: 10.1021/acs.langmuir.5b04334
- PIETER ESPEEL ET AL: "One-Pot Multistep Reactions Based on Thiolactones: Extending the Realm of Thiol-Ene Chemistry in Polymer Synthesis", JOURNAL OF THE AMERICAN CHEMICAL SOCIETY, Bd. 133, Nr. 6, 16. Februar 2011 (2011-02-16), Seiten 1678-1681, XP055320067, US ISSN: 0002-7863, DOI: 10.1021/ja1098098
- PIETER ESPEEL ET AL: "One-pot multi-step reactions based on thiolactone chemistry: A powerful synthetic tool in polymer science", EUROPEAN POLYMER JOURNAL., Bd. 62, 1. Januar 2015 (2015-01-01), Seiten 247-272, XP055284256, GB ISSN: 0014-3057, DOI: 10.1016/j.eurpolymj.2014.07.008
- HEREDIA KARINA L ET AL: "In situ preparation of protein-smart polymer conjugates with retention of bioactivity", JOURNAL OF THE AMERICAN CHEMICAL SOCIETY, AMERICAN CHEMICAL SOCIETY, US, Bd. 127, Nr. 48, 7. Dezember 2005 (2005-12-07), Seiten 16955-16960, XP002514531, ISSN: 0002-7863, DOI: 10.1021/JA054482W

## Beschreibung

Die vorliegende Erfindung betrifft ein Verfahren zur Herstellung eines Protein-funktionalisierten Films, bei dem ein Protein an ein Copolymer oder ein Polymer mit einer unhydrolisierten oder hydrolisierten Thiolacton-Funktionalisierung über die vorhandene Funktionalisierung an den Film angebunden wird. Die vorliegende Erfindung betrifft zudem einen entsprechend hergestellten Film.

Die Herstellung dünner enzymhaltiger Polymerschichten ist zahlreich beschrieben und dient u.a. der Stabilisierung des verwendeten Enzyms sowie der Kopplung von enzymatischen Reaktionen mit geeigneten Signalübertragungswegen. Die geringe Dicke solcher Schichten ist vor allem für Anwendungsbereiche interessant, bei denen nur geringe Mengen an aktivem Material benötigt werden, gleichzeitig aber schnelle, nicht durch Diffusionseffekte behinderte Ansprech- bzw. Konktaktzeiten gefordert sind. Zu nennen ist hier vor allem die Sensorik aber auch Bereiche wie die Biokatalyse. Jedoch findet sich bisher kein System, bei dem eine einzige funktionelle Gruppe eine solche Vielzahl an Funktionen im Rahmen der Generierung der enzymhaltigen Schicht übernimmt.

Die reversible Anbindung von Enzymen an eine Polymerschicht über Disulfidbrücken ist in einem Patent von 1979 beschrieben (Patent US 4,176,006), jedoch müssen hier die Thiolgruppen in einem extra Schritt über ein niedermolekulares Mercaptan in die Polymerschicht eingeführt werden.

Verbindungen die sich zur Erzeugung dünner, enzymhaltiger Polymerschichten eignen, müssen eine ganze Reihe von Eigenschaften erfüllen. Sie müssen mit wässrigen Enzymlösungen mischbar bzw. in diesen quellbar sein, um eine hinreichende Beladung mit Enzym zu erreichen. Sie müssen funktionelle Gruppen tragen, die eine feste Anbindung des Enzyms an die Polymerschicht bzw. der Polymerschicht an das Substrat ermöglichen (z.B. kovalent). Eine Quellbarkeit der fertigen Polymerschicht im Arbeitsmedium des Enzyms (i.d.R. wässrige Lösungen) ist gefordert und oft werden Möglichkeiten zur Vernetzung der Polymermatrix angestrebt. Ober allem steht darüber hinaus die Notwendigkeit den Immobilisierungsprozess vom ersten bis zum letzten Schritt proteinkompatibel zu gestalten, d.h. eine Deaktivierung des Enzyms wahrend Prozesses weitgehend zu verhindern.

Eine besondere Bedeutung kommt der Erzeugung ultradünner Schichten durch Selbstassemblierungsvorgange von geeigneten Verbindungen (amphiphile Moleküle, polare Polymere, u.a.) an der Luft-Wasser-Grenzfläche mittels Langmuir-Technik bei, da sie die Möglichkeit bietet, dünne, auf der Wasseroberflache gespreitete Schichten kontrolliert und in gewünschtem Maße zu komprimieren und damit Schichtdicken und -dichten einzustellen. Polymere als verwendetes Material haben dabei gegenüber niedermolekularen, amphiphilen Verbindungen den Vorteil, dass sie mechanisch stabilere Schichten bilden. Verwendbare Polymere müssen allerdings eine limitierte Wasserlöslichkeit aufweisen und strukturell einen gewissen Polaritätsgradienten aufweisen. Üblicherweise wird dies durch den Einsatz von amphiphilen Blockcopolymerstrukturen gelöst, die jedoch in ihrer Herstellung komplizierter sind als etwa Homopolymere oder statistische Copolymere. Grundsätzlich können auch Homopolymere bzw. statistische Copolymere Langmuirfilme ausbilden (einen Polaritätsgradienten gäbe es hier entlang der Struktur einer Wiederholungseinheit), jedoch muss auch hier die limitierte Löslichkeit gewährleistet sein. Da dies aber im Widerspruch steht zu der für die spätere Anwendung geforderten Wasserquellbarkeit der Polymermatrix, muss zusätzlich noch ein Weg gefunden werden, diese nachträglich zu hydrophilisieren.

H. Wang et al. beschreiben in LANGMUIR 2016, 32, 151-158 die Funktionalisierung von Nanoporen dünner Polymer-Filme mit Proteinen. Poren verschiedener Größe werden in Polystyrol und Poly(meth)methylacrylat-Filmen produziert und mit Proteinen funktionalisiert.

Das technische Problem liegt also darin, eine möglichst einfach aufgebaute und damit leicht zugängliche Polymerstruktur zu finden, die im Zuge der Bildung von ultradünnen, enzymhaltigen Schichten auf einem festen Substrat oder an der Luft-Wasser-Grenzfläche mittels Langmuir-Technik eine Vielzahl von Funktionen übernehmen kann: Hydrophobisierung des Polymers mit nachträglicher Möglichkeit zur Hydrophilisierung, kovalente Anbindung des Enzyms an die Polymermatrix, kovalente Anbindung der Polymermatrix an das gewünschte Substrat, nachträgliche Vernetzung der gebildeten Schicht.

Ein Verfahren, welches eine einfache, leicht zugangliche polymere Struktur einsetzt, die sämtliche oben angesprochenen Funktionen vereint und auch noch zum Teil aus biogenen Rohstoffen gewonnen werden kann, existiert bisher nicht. Funktionelle Gruppen in den Polymeren oder Polymerprecursoren übernehmen in der Regel jeweils nur eine Funktion. Zur kovalenten Anbindung von Enzymen werden in der Regel funktionelle Einheiten wie Oxiran-, Carboxyl- oder Aminogruppen eingesetzt wobei z.B. bei letzterem noch zusätzliche, mitunter giftige Linker oder Cokomponenten zur Anbindung des Enzyms notwendig sind. Oft wird das Enzym auch lediglich in einer Polymermatrix ohne kovalente Anbindung physikalisch eingeschlossen. Auch die Vernetzung der Polymermatrix erfolgt meist über zusätzlich einzusetzende Vernetzer.

Um polymerbasierte Schichten an der Luft-Wasser-Grenzfläche zu erzeugen verlässt man sich üblicherweise auf amphiphile Blockstrukturen, wobei das Enzym dann in der Regel auch nur physisorbiert, jedoch nicht kovalent angebunden ist. Etwaig vorhandene funktionelle Gruppen übernehmen auch in diesen Fällen in der Regel nur eine Funktion.

Ausgehend hiervon ist es somit Aufgabe der vorliegenden Erfindung, ein Verfahren zur Herstellung eines Protein-funktionalisierten Films anzugeben, das die oben angesprochenen Nachteile vermeidet. Zudem ist es die Aufgabe der vorliegenden Erfindung, einen Protein-funktionalisierten Film bereitzustellen.

Diese Aufgabe wird hinsichtlich eines Verfahrens mit den Merkmalen des Patentanspruchs 1 bezüglich eines Protein-funktionalisierten Films mit den Merkmalen des Patentanspruchs 15 gelöst. Die jeweilig abhängigen Patentansprüche stellen dabei vorteilhafte Weiterbildungen dar.

Die Erfindung betrifft demnach ein Verfahren zur Herstellung eines Protein-funktionalisierten Films, bei dem ein Film, enthaltend mindestens ein Copolymer oder Polymer mit einer unhydrolysierten und/oder hydrolysierten Thiolacton-Funktionalisierung sowie mindestens ein Protein erzeugt oder bereitgestellt, und das mindestens eine Protein über die unhydrolisierte oder hydrolisierte Thiolacton-Funktionalisierung kovalent an das mindestens eine Copolymeren oder Polymere angebunden wird.

Kennzeichnendes Merkmal des erfindungsgemäßen Verfahrens ist, dass ein spezifisches Copolymer bzw. Polymer eingesetzt wird, das eine (unhydrolisierte oder hydrolisierte) Thiolacton-Funktionalisierung aufweist. Bevorzugt ist die Thiolacton-Funktionalisierung dabei über ein entsprechendes Monomer in das Copolymer bzw. Polymer eingearbeitet. Die Thiolacton-Funktionalisierung kann allerdings auch in einer Seitenkette des Copolymeren bzw. Polymeren vorliegen, beispielsweise für den Fall, dass es sich bei den Copolymeren bzw. Polymeren um Pfropfpolymere handelt. Lediglich das Vorhandensein der Thiolacton-Funktionalisierung ist zur kovalenten Anbindung des Proteins erfindungswesentlich.

Das Copolymer und/oder Polymer kann dabei entweder ausschließlich unhydrolisierte oder ausschließlich hydrolisierte Thiolacton-Funktionalisierungen umfassen. Ebenso ist es möglich, dass das Copolymer bzw. Polymer beide Arten der Funktionalisierung aufweist.

Die Erfindung zeichnet sich insbesondere durch die folgenden Vorteile aus:
- Eine funktionelle Gruppe - die bis zu 5 Funktionen erfüllt:
   - Kovalente Anbindung der Polymerschicht an das Substrat
   - Kovalente Anbindung des Proteins, insbesondere eines Enzyms an die Polymerschicht
   - Vernetzung der Polymerschicht
   - Steuerung der Hydrophilie/Hydrophobizitat des Polymers
   - Bereitstellung von Thiolgruppen zur reversiblen Anbindung von Enzymen über Bildung von Disulfidbrücken bzw. zur Fixierung der Polymermatrix auf Metalloberflächen
- Leichte Zugänglichkeit dieser funktionellen Gruppe aus biobasierten Rohstoffquellen
- Verwendung eines leicht zuganglichen statistischen Copolymers hergestellt durch radikalische Polymerisation, Vermeidung komplizierter amphiphiler Block- bzw. multifunktioneller Strukturen

Der Hauptvorteil liegt darin, dass eine funktionelle Gruppe, die leicht zuganglich ist und ebenso leicht in eine Polymerkette eingebaut werden kann, eine Vielzahl von Funktionen übernimmt die im Zuge der Bildung dünner enzymhaltiger Schichten auf verschiedenen Substraten von Bedeutung sind. Dadurch wird die Verwendung von komplexen, multifunktionellen Polymerstrukturen oder Multikomponentensystemen vermieden. Gleichzeitig handelt es sich bei dieser funktionellen Gruppe um eine Verbindung, die z. B. aus biobasierten Rohstoffquellen gewonnen wird. Die Möglichkeit die Thiolactongruppen in verschiedenste Polymere einzubauen führt darüber hinaus zu einer vergleichsweise flexiblen Immobilisierungsplattform. Auch wenn das Verfahren letztlich für jedes Enzym individuell getestet werden muss, so ist es grundsätzlich auf jedes Protein anwendbar, welches z. B. mindestens eine von außen zugängliche Lysineinheit trägt.

Der Film aus den Copolymeren oder Polymeren kann dabei in situ erzeugt werden, indem der Film beispielsweise aus einer Lösung des Copolymeren oder Polymeren abgeschieden wird. Alternativ kann der Film des thiolactonfunktionalisierten Copolymeren oder Polymeren auch bereits vorab gefertigt und für die Zwecke des erfindungsgemäßen Verfahrens bereitgestellt werden.

Gemäß einer bevorzugten Ausführungsform wird der Film allerdings in situ erzeugt und hierbei insbesondere direkt auf einem Substrat oder mittels Langmuir-Schaefer-Technik erzeugt.

Eine bevorzugte Ausführungsform des Erzeugens des Films auf einem Substrat umfasst hierbei das Rakeln, Spin-Coating und/oder Sprühauftrag aus einer Lösung des mindestens einen Copolymeren und/oder Polymeren, bevorzugt aus (schwach) saurer oder neutraler Lösung, insbesondere aus einer Lösung mit einem pH-Wert von 5 bis 7. Zur Herstellung der Lösungen der Copolymeren und/oder Polymeren werden dabei Lösungsmittel verwendet, die in der Lage sind, die jeweiligen Copolymeren bzw. Polymeren zu lösen.

Eine bevorzugte Ausführungsform zur Erzeugung des Films mittels Langmuir-Schaefer-Technik sieht ein Spreiten einer Lösung des mindestens einen Copolymeren und/oder Polymeren in einem flüchtigen, nicht mit Wasser mischbaren Lösungsmittel, insbesondere Chloroform, auf der Oberfläche von Wasser oder einer wässrigen Lösung vor.

Für den Fall, dass der Film des Copolymeren oder Polymeren auf einem Substrat abgeschieden wird, ist das Substrat bevorzugt ausgewählt aus der Gruppe bestehend aus Polymerfilmen oder -membranen, bevorzugt mit Aminogruppen oberflächenfunktionalisierten Polymerfilmen oder Membranen, insbesondere mit Aminogruppen oberflächenfunktionalisierten Filmen oder Membranen aus Polyacrylnitril, Polydimethylsiloxan, cellulosebasierten Polymeren, Polyvinylalkoholen, Poly(hydroxyalkylacrylate), insbesondere Poly(2-hydroxyethylacrylat), Poly(hydroxymethylacrylat) sowie Copolymere hiervon sowie anorganischen Substraten, bevorzugt mit Aminogruppen oberflächenfunktionalisierten anorganischen Substraten, insbesondere mit Aminogruppen oberflächenfunktionalisierten anorganischen Siliziumwafer, Glassubstrate oder Metallsubstrate, insbesondere Münzmetallsubstraten oder mit Münzmetallen beschichteten Substraten. Besonders bevorzugt als Münzmetall ist dabei Gold. Metalle, die eine kovalente Bindung mit Thiolgruppen eingehen können, beispielswiese die zuvor genannten Münzmetalle, insbesondere Gold brauchen dabei nicht notwendigerweise mit Aminogruppen oberflächenfunktionalisiert sein.

Über die vorhandenen Thiolacton-Einheiten im mindestens eine Copolymeren kann somit ein kovalentes Anbinden des Films an ein ggf. vorhandenes Substrat erfolgen. Für den Fall, dass das Substrat eine oberflächlich vorhandene Aminofunktionalisierung aufweist, können diese Aminogruppen in Analogie zu den Aminogruppen des anzubindenden Proteins mit den Thiolactongruppen reagieren. Andererseits kann alternativ oder zusätzlich hierzu auch ein kovalentes Anbinden des Copolymeren oder Polymeren an das Substrat direkt über freie SH-Gruppen der hydrolysierten Thiolactoneinheit erfolgen, beispielsweise im Falle von Substraten aus Münzmetallen oder mit Münzmetallen beschichteten Substraten.

Das Protein kann auf verschiedene Art und Weise mit den Copolymeren oder Polymeren vor Erzeugung der kovalenten Bindung in Kontakt gebracht werden und dadurch in den Film des mindestens einen Copolymeren oder Polymeren eingearbeitet werden.

Beispielsweise kann das Protein nach Erzeugung oder Bereitstellung des Films des mindestens einen Copolymeren oder Polymeren auf die Oberfläche des Films, insbesondere durch, Spin-Coating und/oder Sprühauftrag einer Lösung des mindestens einen Proteins aufgetragen werden. Bevorzugt wird hierbei der Film des mindestens einen Copolymeren oder Polymeren mit dem mindestens einen Protein durchdrungen, d.h. das Protein penetriert in den Film des Copolymeren oder Polymeren.

Alternativ oder additiv hierzu ist es ebenso möglich, dass das mindestens eine Protein mit dem mindestens einen Copolymeren und/oder Polymeren vermischt wird und aus dieser Mischung der Film des mindestens einen Copolymeren und/oder Polymeren erzeugt wird. Beispielsweise kann das Copolymere und/oder Polymere zusammen mit dem mindestens einen Protein in eine Lösung gebracht werden und aus dieser Lösung der Film erzeugt werden. Insbesondere bei dieser Ausführungsform ist das mindestens eine Protein homogen innerhalb des Films aus mindestens einem Copolymeren oder Polymeren verteilt.

Im Falle des Erzeugens des Films des mindestens einen Copolymeren und/oder Polymer an der Luft-Wasser-Grenzfläche kann das mindestens eine Protein mittels Langmuir-Schaefer-Technik durch Aufgabe des Proteins in die wässrige Subphase, vor, während und/oder nach Erzeugen des Films des mindestens einen Copolymeren und/oder Polymeren und Ad- und/oder Absorption des mindestens einen Proteins an und/oder in den Film des Copolymeren und/oder Polymeren eingearbeitet werden.

Erfindungsgemäße unhydrolysierte Thiolacton-Funktionalisierungen sind hierbei ausgewählt aus der Gruppe aus Resten mit der nachfolgend abgebildeten allgemeinen Formel I

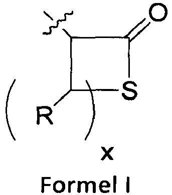

Eine hydrolisierte Thiolacton-Funktionalisierung ist dabei ausgewählt aus der Gruppe aus Resten mit der nachfolgend abgebildeten allgemeinen Formel II

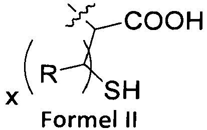

In den voranstehend beschriebenen Formeln I und II bedeuten jeweils unabhängig voneinander
- R: Wasserstoff oder einen linearen oder verzweigten Alkylrest mit 1 bis 8 Kohlenstoffatomen und
- x: 1 bis 6.

Gemäß einer bevorzugten Ausführungsform ist vorgesehen, dass der Rest R Wasserstoff bedeutet und x 2 oder 3 ist, insbesondere 2 ist.

Eine weitere bevorzugte Ausführungsform sieht vor, dass das Copolymere Wiederholungseinheiten beinhaltet oder das Polymere aus Wiederholungseinheiten gebildet ist mit der nachfolgenden allgemeinen Formel III (unhydrolisiert) oder IV (hydrolisiert) 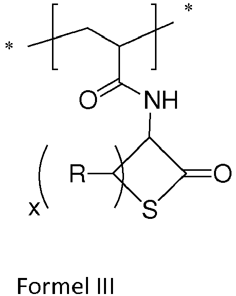 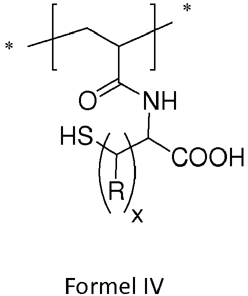 wobei jeweils unabhängig voneinander
- R: Wasserstoff oder einen linearen oder verzweigten Alkylrest mit 1 bis 8 Kohlenstoffatomen und
- x: 1 bis 6
bedeuten und bevorzugt R Wasserstoff und x 2 oder 3 ist.

Das Copolymere kann hierbei zudem Wiederholungseinheiten beinhalten, die nicht Thiolacton-funktionalisiert sind und insbesondere der nachfolgend abgebildeten allgemeinen Formel genügen: 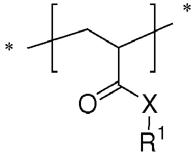 wobei jeweils unabhängig voneinander
- X: NH, O oder NR¹ und
- R¹: einen linearen oder verzweigten Alkylrest mit 1 bis 8 Kohlenstoffatomen oder Wasserstoff bedeuten.

R¹ ist hierbei insbesondere ein Isopropyl-Rest , X insbesondere die Funktionalisierung NH.

Gemäß einer insbesondere bevorzugten Ausführungsform ist das Copolymere aus den beiden folgenden Wiederholungseinheiten a) und b) gebildet ist:
a) 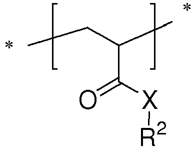 wobei jeweils unabhängig voneinander
   - X: NH,
   - R²: einen der nachfolgenden Reste 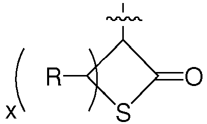 oder 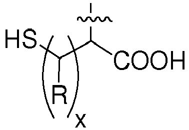 wobei jeweils unabhängig voneinander
   - R: Wasserstoff oder einen linearen oder verzweigten Alkylrest mit 1 bis 8 Kohlenstoffatomen und
   - x: 1 bis 6
   bedeuten, und
b) 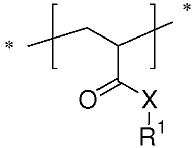 wobei jeweils unabhängig voneinander
   X NH, O oder NR¹ und
   R¹ einen linearen oder verzweigten Alkylrest mit 1 bis 8 Kohlenstoffatomen oder Wasserstoff bedeuten,
wobei die Wiederholungseinheiten a) und b) statistisch verteilt im Copolymeren vorliegen.

Beim zuvor genannten bevorzugten Copolymeren ist es insbesondere vorteilhaft, wenn der molare Anteil der Wiederholungseinheiten a), bezogen auf die Gesamtheit der Wiederholungseinheiten a) + b) 5 bis 50 mol-%, bevorzugt 10 bis 40 mol-%, besonders bevorzugt 15 bis 30 mol% beträgt.

Bevorzugt ist das Protein, das kovalent an das Copolymer bzw. Polymer angebunden wird, ein Enzym, insbesondere ein Enzym ausgewählt aus der Gruppe bestehend aus Aldolasen, Hydrolasen, wie z.B. Lipasen, Proteasen, Amidasen, Acylasen, Nitrilasen, Dehalogenasen, Isomerasen, Transferasen, oder ein funktionelles Protein insbesondere Kanalproteine und Antikörper sowie Kombinationen hiervon.

Der Film kann beispielsweise mit 0,01 bis 50 Gew.-%, bevorzugt 1 bis 20 Gew.% des mindestens einen Proteins beladen werden.

Eine weitere bevorzugte Ausführungsform sieht vor, dass zur kovalenten Anbindung des Proteins an das mindestens eine Copolymer oder Polymer mit einer unhydrolysierten Thiolacton-Funktionalisierung der im ersten Schritt erzeugte Protein enthaltende Film über 0,1 bis 24 h, bevorzugt 0,1 bis 8h h, insbesondere 0,1 bis 2 h gelagert wird.

Die zuvor beschriebene Lagerung wird dabei bei zumindest einem der nachfolgenden Parameter ausgeführt: Bei Temperaturen von 0 bis 30 °C, bevorzugt 0 bis 10 °C und/oder bei einem pH von 7,5 bis 12, bevorzugt 8 bis 10 und/oder unter Einwirkung eines Oxidationsmittels, beispielsweise Wasserstoffperoxid.

Insbesondere bei den zuvor genannten Nachbehandlungsschritten findet eine Vernetzung des mindestens einen Copolymeren und/oder Polymeren statt, indem noch vorhandene Thiolacton-Funktionalitäten hydrolysiert und vorhandene freie oder durch die Hydrolyse entstandene Thiolfunktionalitäten untereinander Disulfidbrücken ausbilden.

Zudem ist insbesondere über die beschriebene Nachbehandlung ebenso eine kovalente Anknüpfung des mindestens einen Copolymeren und/bzw. Polymeren an ein ggf. vorhandenes Substrat möglich, indem beispielsweise eine Aminogruppe eines oberflächlich Aminogruppen-funktionalisierten Substrats mit einem noch vorhandenen Thiolacton reagiert oder eine freie Thiolgruppe mit einem Thiolgruppen-affinen Substrat, bevorzugt einem Metall oder mit Metall beschichteten Substrat, insbesondere einem Münzmetall oder mit Münzmetall beschichteten Substrat, beispielsweise ein Goldsubstrat oder ein mit Gold beschichtetes Substrat unter Ausbildung einer kovalenten Bindung reagiert.

Alternativ hierzu ist es ebenso bevorzugt, dass zur kovalenten Anbindung des Proteins an das mindestens ein Copolymer oder Polymer mit einer hydrolysierten Thiolacton-Funktionalisierung der im ersten Schritt erzeugte Protein enthaltende Film unter oxidierenden Bedingungen behandelt wird, wobei Disulfidbrücken zwischen proteineigenen Thiolgruppen und den Thiolgruppen des hydrolysierten Thiolactons erzeugt werden.

Die Erfindung betrifft zudem einen Protein-funktionalisierten Film, enthaltend mindestens ein Copolymer und/oder Polymer, an das über einen Spacer, ausgewählt aus den nachfolgend abgebildeten allgemeinen Formeln V und VI 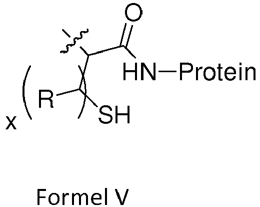 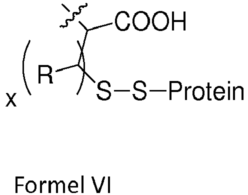 wobei jeweils unabhängig voneinander
R Wasserstoff oder einen linearen oder verzweigten Alkylrest mit 1 bis 8 Kohlenstoffatomen und
x 1 bis 6
bedeuten,
mindestens ein Protein kovalent angebunden ist.

Die freien SH-Gruppen in Formel V können zum Teil oder insgesamt durch den Nachbehandlungsschritt Disulfidbrücken zu weiteren freien SH-Gruppen der Formel V und/oder zu Thiolgruppen von Proteinen ausbilden und so den Polymerfilm vernetzend stabilisieren.

Zudem kann auch ein kovalentes Anbinden des Substrates über die Thiolactonfunktionalisierung gemäß den weiter oben stehenden Mechanismen erfolgen.

Sämtliche zuvor genannten bevorzugten Ausführungsformen, die im Zusammenhang mit dem Verfahren genannt sind, insbesondere hinsichtlich der einsetzbaren bzw. angebundenen Proteine, der bevorzugten Ausführungsformen hinsichtlich der zur Anbindung verwendeten funktionellen Gruppierungen gelten sowie möglicher Substrate uneingeschränkt ebenso für den erfindungsgemäßen Protein-funktionalisierten Film.

Der erfindungsgemäße Protein-funktionalisierte Film zeichnet sich insbesondere durch eine Dicke von 5 nm bis 500 nm aus.

Der Film kann dabei wie vorstehend beschrieben ebenso auf einem Substrat, das zuvor bereits eingehender definiert wurde, angeordnet sein.

Eine weiter bevorzugte Ausführungsform sieht vor, dass der Gehalt des mindestens einen Proteins, bezogen auf die Gesamtmasse des Films von 0,01 bis 50 Gew%, bevorzugt 1 bis 20 Gew.% beträgt.

Technische Anwendungsmöglichkeiten der vorliegenden Erfindung sind insbesondere die Immobilisierung von Enzymen auf Membransubstraten fur biokatalystische Anwendungen. Hier geht es vor allem um die Möglichkeit, die Enzymstabilität zu erhöhen, aufwendige Reinigungsschritte zu vermeiden sowie kontinuierliche Prozessführungen zu erlauben. Als Beispiel sei hier die oben näher beschriebene beispielhaft erwähnte Immobilisierung einer Aldolase erwähnt, ein Enzym, welches bei der Herstellung von pharmazeutischen Wirkstoffen Verwendung findet. Zur Herstellung solcher Membranen können im Prinzip etablierte, skalierbare Membranziehverfahren angewendet werden. Die Möglichkeit zur Regenerierung der enzymaktiven Membran, d.h. des Neubeladens dieser mit Enzym ohne die Notwendigkeit, die Membran komplett austauschen zu müssen, dürfte für viele technische Anwendungen einen wichtigen Pluspunkt darstellen. Ein weiteres relevantes Anwendungsgebiet wäre in der Sensorik zu suchen. Glucosesensoren beispielsweise basieren oft auf Glucose-Oxidase die in dünnen Schichten immobilisiert ist.

Die vorliegende Erfindung wird anhand der nachfolgenden Ausführungen näher beleuchtet, ohne die Erfindung auf die speziell dargestellten Parameter zu beschränken.

Der Gegenstand der vorliegenden Erfindung wird nachfolgend insbesondere am Beispiel der Nutzung von thiolactonhaltigen Copolymeren für verschiedene Verfahren zur Erzeugung dünner, enzymhaltiger Polymerschichten bzw. - filme auf variierenden Substraten näher erläutert.

Diese Schichten bzw. Filme können dabei entweder direkt auf dem gewünschten Substrat durch Techniken wie Spin-Coating und Sprühauftrag generiert oder zunächst an der Luft-Wasser-Grenzfläche mit anschließendem Übertrag auf das Substrat mittels Langmuir-Schaefer-Technik gebildet werden. Zentrales Element sind dabei die γ-Butyrothiolactoneinheiten des verwendeten Copolymers, die bevorzugt als statistisch verteilte Substituenten entlang der Polymerkette angeordnet sind.

Diese nehmen mehrere, für das jeweilige Immobilisierungsverfahren essentielle Funktionen ein, deren Kombination üblicherweise nur durch Verwendung strukturell komplexer Verbindungen erreicht werden kann.

Gleichzeitig sind γ-Butyrothiolactonderivate als Produkte der intramolekularen Kondensationsreaktion von Methionin oder Homocystein leicht aus biologischen Rohstoffquellen zu erschließen.

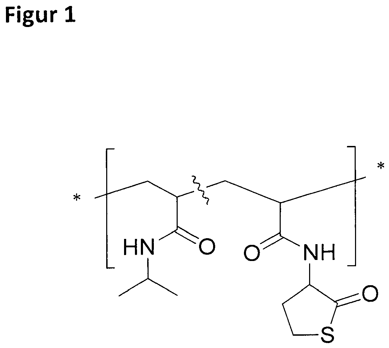

Die Herstellung der dünnen, enzymhaltigen Polymerschicht geschieht bevorzugt in zwei Stufen. Zunächst wird die Schicht selbst auf einem passenden Substrat generiert wobei das Enzym hier zunächst nur physikalisch in die Polymerschicht eingebunden ist. In einem Nachbehandlungsschritt wird dann die feste Verbindung aller Komponenten sowie die Vernetzung und u.U. Hydrophilisierung der Polymermatrix gewährleistet. Für die Grundstruktur des verwendeten Polymers kommen verschiedene Polymerklassen in Frage, wie etwa (meth)acrylamid-, oder (meth-)acrylatbasierte Systeme. Diese sind leicht durch radikalische Copolymerisation des Hauptmonomeren mit einem analogen, thiolactonfunktionalisierten Comonomer synthetisierbar. Geht man von N-Isopropylacrylamid als Hauptmonomereinheit aus (siehe Figur 1, in der die Grundstruktur eines thiolactonhaltigen, statistischen Copolymers auf N-Isopropylacrylamid-Basis dargestellt ist), so kommt man durch einen genügend hohen Anteil an Thiolactonacrylamid-Comonomer zu einem Polymer welches in der Lage ist, definierte Langmuirfilme auszubilden und dabei hinreichend hydrophob ist, so dass dieser Film an der Luft-Wasser-Grenzflache Ober einen längeren Zeitraum hinweg stabil bleibt. Greift man andererseits auf N,N-Dimethylacrylamid als Hauptmonomereinheit zurück, können wiederum Polymere erzeugt werden, die auch bei höheren Thiolactongehalten noch wasserlöslich sind, was vor allem für das Verfahren Ober den direkten Auftrag der Schicht auf ein Substrat wichtig ist.

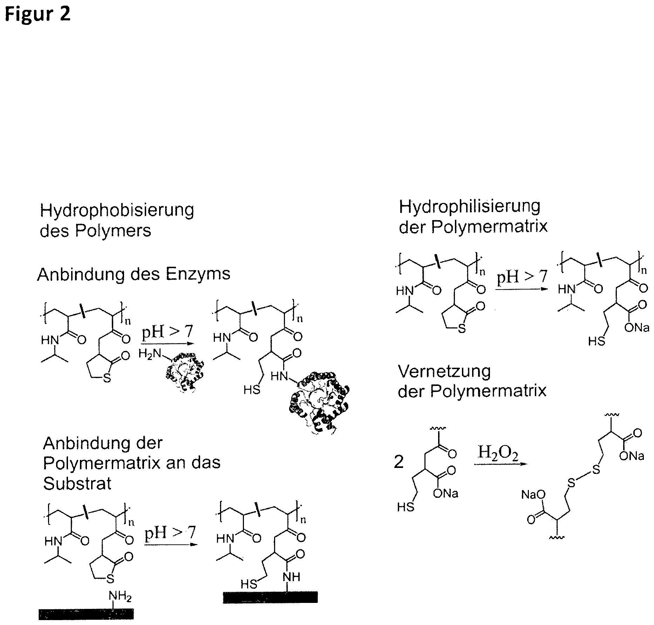

Sämtliche Funktionen, die die Thiolactoneinheiten im Zuge des Immobilisierungsverfahrens einnehmen, sind in Figur 2 dargestellt. Ersichtlich sind die von den Thiolactongruppen des eingesetzten Polymers übernommenen Funktionen im Zuge der Generierung enzymhaltiger, dünner Polymerschichten auf variierenden Substraten.

### A) Generierung der dünnen, enzymhaltigen Schicht

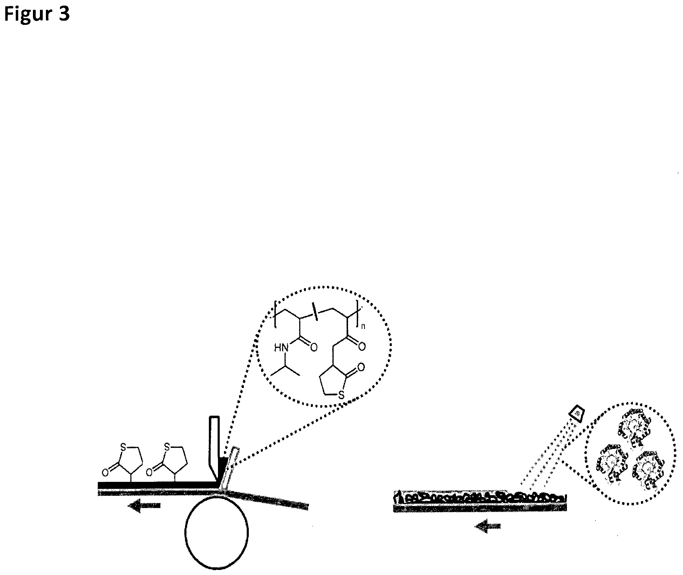

Der Direktauftrag der Polymerschicht auf ein Substrat, beispielsweise einer Polyacrylnitrilmembran die mit Aminogruppen oberflächenfunktionalisiert ist, kann durch Rakeln, Spin-Coating oder Sprühauftrag aus leicht saurer Lösung erfolgen. Die Beladung mit Enzym erfolgt dann ebenfalls durch Sprühauftrag. (Figur 3). In Figur 3 links dargestellt ist die Applikation der Polymerschicht durch Rakeln; rechts die Beladung der aufgetragenen Polymerschicht mit Enzymlösung durch Sprühauftrag.

Alternativ werden Polymer und Enzym in einer Lösung gemischt und dann gemeinsam aufgetragen.

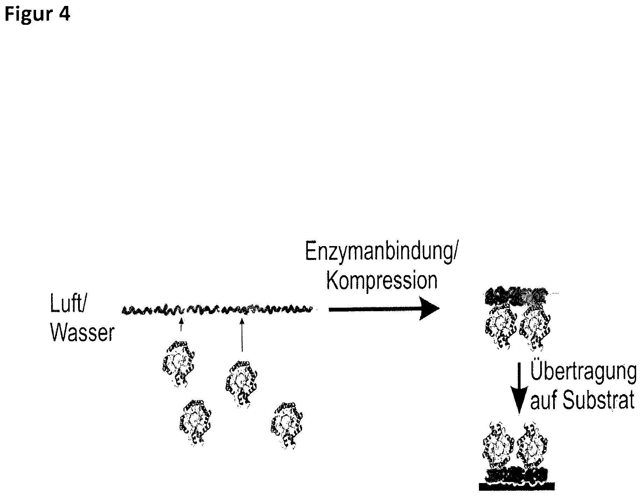

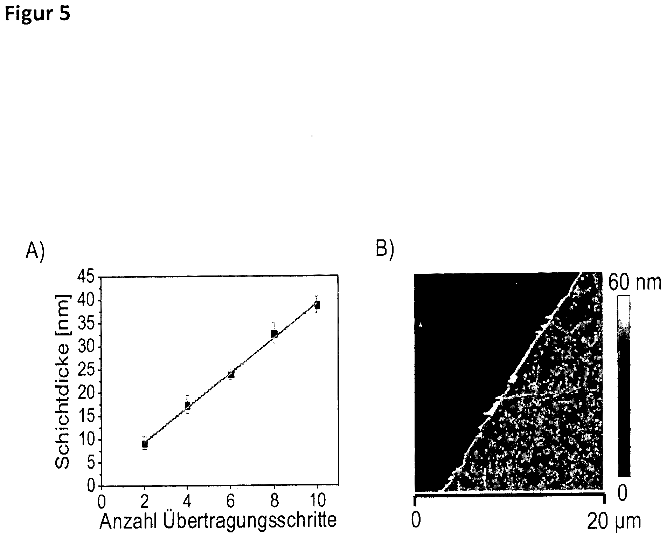

Im Falle der Filmerzeugung an der Luft-Wasser-Grenzfläche mittels Langmuir-Schaefer wird der Polymerfilm erzeugt, indem eine Lösung des Polymeren in Chloroform auf der Wasseroberfläche gespreitet wird. Nach Verdampfen des Lösungsmittels kann der Film durch Bewegung der Barrieren der Langmuir-Filmwaage beliebig komprimiert werden bevor im Anschluss das zu immobilisierende Protein, hier beispielhaft eine Aldolase, in die Subphase injiziert wird. Nach einer festgelegten Wartezeit (etwa 1-2 Stunden), während der das Enzym an den Polymerfilm adsorbiert, wird dieser nach einer eventuellen, weiteren Komprimierung und damit Verdichtung durch einfaches Auftippen eines passenden, aminfunktionalisierten Substrats auf die Wasseroberfläche auf jenes Substrat übertragen (siehe Figur 4, die schematisch die Erzeugung einer ultradünnen, enzymhaltigen Polymerschicht mittels Langmuir-Schaefer-Technik zeigt). Durch mehrfache Wiederholung des Übertragungsschritts kann kontrolliert ein ultradünner Film mit gewünschter Schichtdicke aufgebaut werden (siehe Figur 4). Dargestellt ist hierbei der kontrollierte Aufbau einer ultradünnen Schicht eines *N*-isopropylacrylamid (NIPAAm)-basierten Polythiolactones aus Figur 1 auf einem geeigneten Substrat (hier: aminofunktionalisierte Silizium-Wafer) mittels Langmuir-Schaefer; A) Schichtdicke in Abhängigkeit von der Anzahl an Übertragungsschritten; B) AFM Topographiebild einer Polymerschicht entstanden aus aufeinanderfolgenden 8 Übertragungsschritten.

### B) Nachbehandlungsschritt

Beispielhaft wird nachfolgend die Nachbehandlung einer durch Langmuir-Schaefer erzeugten aldolasehaltigen Polymerschicht beschrieben. Die Nachbehandlung von Filmen, die durch Direktauftrag gewonnen wurden erfolgt jedoch völlig analog. Im Anschluss an die Filmerzeugung und -übertragung wird das beschichtete Substrat in einem leicht basischen Puffer (pH = 9) bei 5 °C für einige Stunden gelagert, um die Anbindung des Enzyms an das Polymer sowie die Fixierung des Polymers an das Substrat über die Thiolactoneinheiten zu forcieren (siehe hierzu auch Figur 2). Gleichzeitig wird ein weiterer Teil der Thiolactone hydrolysiert, wodurch die Polymermatrix hydrophilisiert wird. Die Umsetzung der Thiolactone bedingt wiederum die Freisetzung von Thiolgruppen, die unter oxidativen Bedingungen Disulfidbrücken ausbilden können und damit eine zusätzliche Vernetzung des Polymerfilms bewirken. Um letzteres in ausreichendem Maße zu gewährleisten, setzen wir geringe Mengen Wasserstoffperoxid zu. Die erfolgreiche Hydrophilisierung kann durch Kontaktwinkelmessungen und AFM-Studien nachgewiesen werden. Steht für die Anbindung der Polymermatrix an das Substrat kein entsprechend aminfunktionalisiertes Material zur Verfügung, kann diese auch prinzipiell über die freigesetzten Thiolgruppen erfolgen. Diese Anbindungsstrategie kommt beispielsweise für Metalloberflächen in Frage.

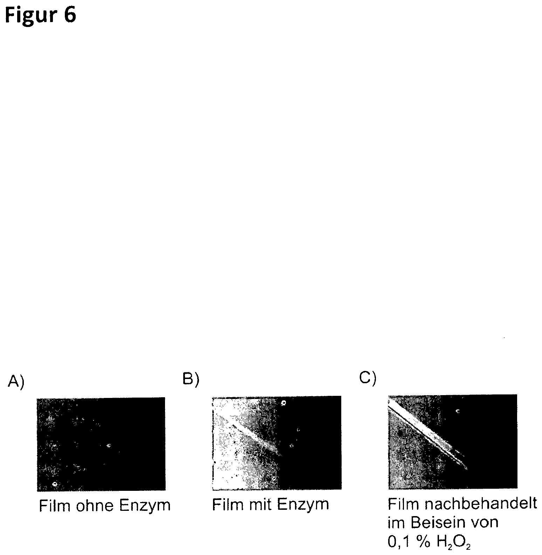

Durch Verwendung von im Vorfeld mit einem Fluoreszenzfarbstoff markiertem Enzym konnte gezeigt werden, dass die an Luft-Wasser-Grenzfläche erzeugten Filme tatsächlich enzymhaltig sind, wobei das Ausmaß der anfänglichen Komprimierung des Films die immobilisierte Menge an Enzym direkt beeinflusst. Da das verwendete Enzym wie die meisten Proteine ein multifunktionelles Amin darstellt, bedingt bereits dessen Anbindung an die Thiolactoneinheiten der Polymermatrix eine Vernetzung derselbigen. Deutlich wird dies anhand von Mikroskopiebildern, die zeigen, dass die Nachbehandlung der übertragenen Schichten diese deutlich intakter zurücklässt wenn Enzym anwesend ist, während ein Großteil des Materials in Abwesenheit von Enzym während der Nachbehandlung abgewaschen wird (siehe hierzu Figur 6, in der optische Mikroskopiebilder von dünnen, NIPAAm-basierten Polythiolactonschichten auf Silizium-Wafern, erzeugt mittels Langmuir-Schaefer, nach der Behandlung mit schwach basischem Kaliumphosphatpuffer (pH = 9) dargestellt sind). Der Kontrast zwischen den Bereichen mit und denen ohne Film korreliert direkt mit der Schichtdicke und damit der Menge an Material welches nach dem Nachbehandlungsschritt übriggeblieben ist. Grundsätzlich bleibt jedoch noch mehr Material erhalten, wenn H₂0₂ zur Ausbildung der Disulfidbrücken eingesetzt wird.

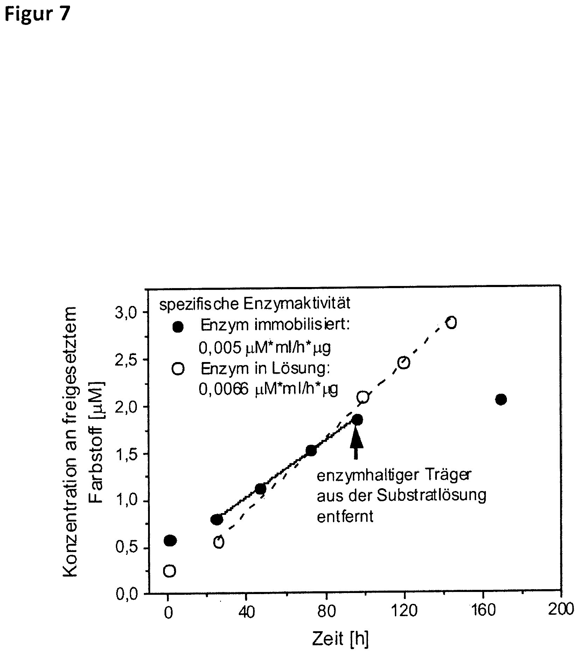

Die übertragenen Schichten zeigen enzymatische Aktivität (Figur 7), wobei im Zuge der Messungen ein Herausdiffundieren des Enzyms nicht oder nur zu einem geringen Teil zu beobachten ist. In Figur 7 ist ein Aktivitatsassay für 2-Deoxy-5-phosphatealdolase (DERA) basierend auf der Freisetzung eines fluoreszierenden Farbstoffs im Zuge der enzymatischen Umsetzung eines entsprechenden Substrats gezeigt. Die jeweilige Konzentration an Enzym (immobilisiert und in Lösung) betrug ca. 3 µg/mL (bezogen auf das Volumen der Substratlösung). Mit Hilfe von Fluoreszenzmessungen mit denen sich die jeweilige Menge an immobilisiertem Enzym abschätzen lasst, kann aus den Ergebnisse der Aktivitätsstudien geschlossen werden, dass die spezifische Aktivität des immobilisierten Enzyms und die des entsprechenden Enzyms in Lösung von der Größenordnung her ähnlich sind. Ein massiver Verlust an Enzymaktivität wird durch die erfindungsgemäße Immobilisierungsstrategie effektiv vermieden.

### C) Modifiziertes Protokoll zur Herstellung regenerierbarer Membranen

Eine leichte Modifikation des Verfahrens zum Direktauftrag der enzymhaltigen Polymerschicht auf ein Substrat gibt uns die Möglichkeit regenerierbare enzymhaltige Schichten aufzubauen. Hier wird zunächst das Polymer allein auf die unter A) beschriebene Weise auf ein Substrat aufgebracht. Bevor die Beladung mit Enzym erfolgt wird nun jedoch zunächst eine Hydrolyse der Thiolactoneinheiten durchgeführt. Wichtig dabei ist, dass die Hydrolyse vollständig ist damit später kein Enzym irreversibel gemäß Figur 3 an die Polymerschicht anbinden kann. Die Anbindung erfolgt stattdessen durch Generierung von Disulfidbrücken zwischen enzymeigenen und den im Hydrolyseschritt freigesetzten polymereigenen Thiolgruppen unter oxidativen Bedingungen. Diese Bindungen können zu einem späteren Zeitpunkt durch ein geeignetes Reduktionsmittel wieder gespalten werden um so den Weg für eine neuerliche Beladung mit Enzym freizumachen. Die Thiolactoneinheiten dienen hierbei auch weiterhin zur Anbindung der Polymerschicht an das Substrat.

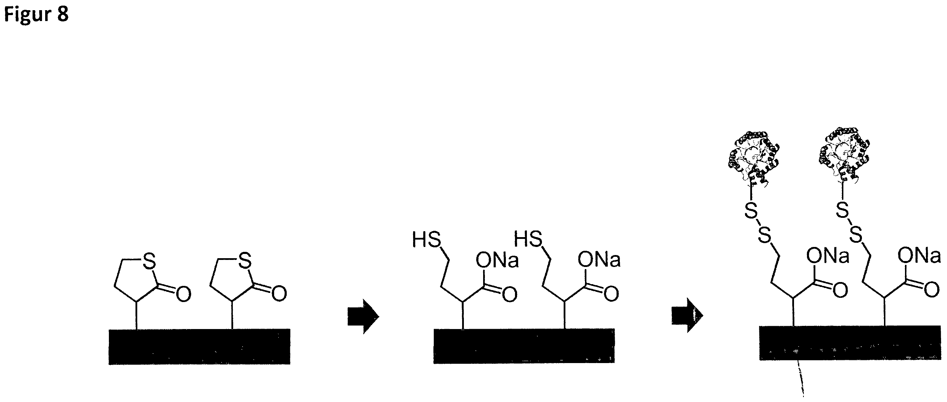

Eine entsprechende Vorgehensweise ist in Figur 8 skizziert, die schematisch ein Verfahren zur Erzeugung regenerierbarer, enzymhaltiger Polymerschichten durch reversible Anbindung des Enzyms an die Polymermatrix über Bildung von Disulfidbrücken darstellt.

## Patentansprüche

1. Verfahren zur Herstellung eines Protein-funktionalisierten Films, bei dem ein Film, enthaltend
mindestens ein Copolymer oder Polymer mit einer unhydrolysierten und/oder hydrolysierten Thiolacton-Funktionalisierung sowie mindestens ein Protein erzeugt oder bereitgestellt, und
das mindestens eine Protein über die unhydrolisierte oder hydrolisierte Thiolacton-Funktionalisierung kovalent an das mindestens eine Copolymeren oder Polymere angebunden wird,
**dadurch gekennzeichnet, dass**
die unhydrolisierte Thiolacton-Funktionalisierung ausgewählt ist aus der Gruppe aus Resten mit der nachfolgend abgebildeten allgemeinen Formel I und die hydrolisierte Thiolacton-Funktionalisierung ausgewählt ist aus der Gruppe aus Resten mit der nachfolgend abgebildeten allgemeinen Formel II 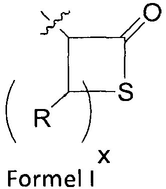 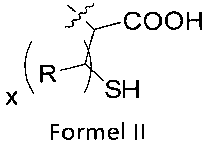 wobei jeweils unabhängig voneinander
R Wasserstoff oder einen linearen oder verzweigten Alkylrest mit 1 bis 8 Kohlenstoffatomen und
x 1 bis 6
bedeuten.

2. Verfahren nach Anspruch 1, **dadurch gekennzeichnet, dass** der Film direkt auf einem Substrat oder mittels Langmuir-Schaefer-Technik erzeugt wird.

3. Verfahren nach vorhergehendem Anspruch, **dadurch gekennzeichnet, dass** die Erzeugung des Films
auf einem Substrat durch Rakeln, Spin-Coating und/oder Sprühauftrag aus einer Lösung des mindestens einen Copolymeren und/oder Polymeren, bevorzugt aus saurer oder neutraler Lösung, insbesondere aus einer Lösung mit einem pH-Wert von 5 bis 7 und/oder mittels Langmuir-Schaefer-Technik durch Spreiten einer Lösung des mindestens einen Copolymeren und/oder Polymeren in einem flüchtigen, nicht mit Wasser mischbaren Lösungsmittel, insbesondere Chloroform, auf der Oberfläche von Wasser oder einer wässrigen Lösung (wässrige Subphase)
erfolgt.

4. Verfahren nach vorhergehendem Anspruch, **dadurch gekennzeichnet, dass** das Substrat ausgewählt ist aus der Gruppe bestehend aus Polymerfilmen oder -membranen, bevorzugt mit Aminogruppen oberflächenfunktionalisierten Polymerfilmen oder Membranen, insbesondere mit Aminogruppen oberflächenfunktionalisierten Filmen oder Membranen aus Polyacrylnitril, Polydimethylsiloxan, cellulosebasierten Polymeren, Polyvinylalkoholen, Poly(hydroxyalkylacrylate), insbesondere Poly(2-hydroxyethylacrylat), Poly(hydroxymethylacrylat) sowie Copoylmere hiervon sowie anorganischen Substraten, insbesondere Metallsubstraten, insbesondere Münzmetallsubstraten oder mit Münzmetall beschichteten Substraten, oder mit Aminogruppen oberflächenfunktionalisierten anorganischen Substraten, insbesondere mit Aminogruppen oberflächenfunktionalisierten anorganischen Siliziumwafer, Glassubstrate.

5. Verfahren nach einem der vorhergehenden Ansprüche, **dadurch gekennzeichnet, dass** das mindestens eine Protein
nach Erzeugung oder Bereitstellen des Films des mindestens einen Copolymeren und/oder Polymeren auf die Oberfläche des Films aufgetragen, insbesondere durch Spin-Coating und/oder Sprühauftrag einer Lösung des mindestens einen Proteins und/oder während der Erzeugung des Films durch Vermischen des mindestens einen Proteins mit dem mindestens einen Copolymeren und/oder Polymeren und/oder
im Falle des Erzeugens des Films des mindestens einen Copolymeren und/oder Polymer mittels Langmuir-Schaefer-Technik durch Aufgabe des Proteins in die wässrige Subphase, vor, während und/oder nach Erzeugen des Films des mindestens einen Copolymeren und/oder Polymeren und Ad- und/oder Absorption des mindestens einen Proteins an und/oder in den Film
in den Film des Copolymeren und/oder Polymeren eingearbeitet wird.

6. Verfahren nach einem der vorhergehenden Ansprüche, **dadurch gekennzeichnet, dass** die unhydrolisierte Thiolacton-Funktionalisierung bevorzugt ausgewählt ist aus der Gruppe aus Resten mit der nachfolgend abgebildeten allgemeinen Formel I und die hydrolisierte Thiolacton-Funktionalisierung bevorzugt ausgewählt ist aus der Gruppe aus Resten mit der nachfolgend abgebildeten allgemeinen Formel II 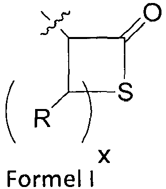 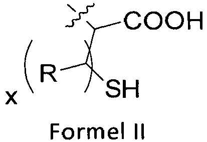 wobei jeweils unabhängig voneinander
R Wasserstoff oder einen linearen oder verzweigten Alkylrest mit 1 bis 8 Kohlenstoffatomen und
x 1 bis 6
bedeuten R Wasserstoff und x 2 oder 3 ist.

7. Verfahren nach einem der vorhergehenden Ansprüche, **dadurch gekennzeichnet, dass** das Copolymere Wiederholungseinheiten beinhaltet oder das Polymere aus Wiederholungseinheiten gebildet ist mit der nachfolgenden allgemeinen Formel III (unhydrolisiert) oder IV (hydrolisiert) 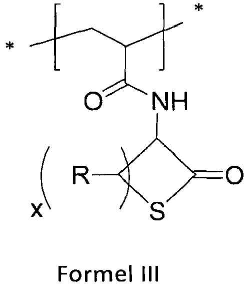 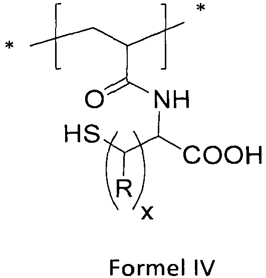 wobei jeweils unabhängig voneinander
R Wasserstoff oder einen linearen oder verzweigten Alkylrest mit 1 bis 8 Kohlenstoffatomen und
x 1 bis 6
bedeuten und bevorzugt R Wasserstoff und x 2 oder 3 ist.

8. Verfahren nach einem der vorhergehenden Ansprüche, **dadurch gekennzeichnet, dass** das Copolymere Wiederholungseinheiten der nachfolgend abgebildeten allgemeinen Formel beinhaltet 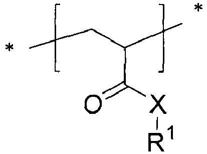 wobei jeweils unabhängig voneinander
X NH, O oder NR¹ und
R¹ einen linearen oder verzweigten Alkylrest mit 1 bis 8 Kohlenstoffatomen oder Wasserstoff
bedeuten.

9. Verfahren nach einem der vorhergehenden Ansprüche, **dadurch gekennzeichnet, dass** das Copolymere aus den beiden folgenden Wiederholungseinheiten a) und b) gebildet ist:
a) 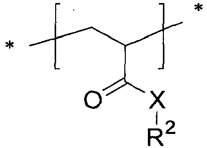 wobei jeweils unabhängig voneinander
X NH,
R² einen der nachfolgenden Reste 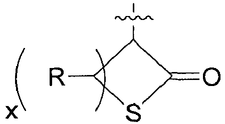 oder 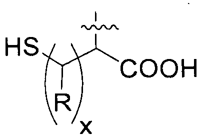
R Wasserstoff oder einen linearen oder verzweigten Alkylrest mit 1 bis 8 Kohlenstoffatomen und
x 1 bis 6
bedeuten, und
b) 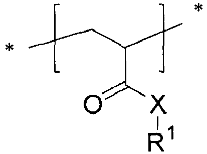 wobei jeweils unabhängig voneinander
X NH, O oder NR¹ und
R¹ einen linearen oder verzweigten Alkylrest mit 1 bis 8 Kohlenstoffatomen oder Wasserstoff
bedeuten, wobei die Wiederholungseinheiten a) und b) statistisch verteilt im Copolymeren vorliegen, wobei insbesondere der molare Anteil der Wiederholungseinheiten a), bezogen auf die Gesamtheit der Wiederholungseinheiten a) + b) 5 bis 50 mol-%, bevorzugt 10 bis 40 mol-%, besonders bevorzugt 15 bis 30 mol% beträgt..

10. Verfahren nach einem der vorhergehenden Ansprüche, **dadurch gekennzeichnet, dass** das Protein ein Enzym ist, insbesondere ein Enzym ausgewählt aus der Gruppe bestehend aus Aldolasen, Hydrolasen, wie z.B. Lipasen, Proteasen, Amidasen, Acylasen, Nitrilasen, Dehalogenasen, Isomerasen, Transferasen, oder ein Protein mit nichtenzymatischer Funktion, insbesondere Kanalproteine und Antikörper sowie Kombinationen hiervon.

11. Verfahren nach einem der vorhergehenden Ansprüche, **dadurch gekennzeichnet, dass** der Film mit 0,01 bis 50 Gew.-%, bevorzugt 1 bis 20 Gew.% des mindestens einen Proteins beladen wird.

12. Verfahren nach einem der vorhergehenden Ansprüche, **dadurch gekennzeichnet, dass** zur kovalenten Anbindung des Proteins an das mindestens eine Copolymer oder Polymer mit einer unhydrolysierten Thiolacton-Funktionalisierung der im ersten Schritt erzeugte Protein enthaltende Film über 0,1 bis 24 h, bevorzugt 0,1 bis 8h h, insbesondere 0,1 bis 2 h gelagert wird.

13. Verfahren nach vorhergehendem Anspruch, **dadurch gekennzeichnet, dass** die Lagerung
bei Temperaturen von 0 bis 30 °C, bevorzugt 0 bis 10 °C und/oder
bei einem pH von 7,5 bis 12, bevorzugt 8 bis 10 und/oder
unter Einwirkung eines Oxidationsmittels, beispielsweise Wasserstoffperoxid,
erfolgt.

14. Verfahren nach einem der Ansprüche 1 bis 11, **dadurch gekennzeichnet, dass** zur kovalenten Anbindung des Proteins an das mindestens ein Copolymer oder Polymer mit einer hydrolysierten Thiolacton-Funktionalisierung der im ersten Schritt erzeugte Protein enthaltende Film unter oxidierenden Bedingungen behandelt wird, wobei Disulfidbrücken zwischen proteineigenen Thiolgruppen und den Thiolgruppen des hydrolysierten Thiolactons erzeugt werden.

15. Protein-funktionalisierter Film, enthaltend mindestens ein Copolymer und/oder Polymer, an das über einen Spacer, ausgewählt aus den nachfolgend abgebildeten allgemeinen Formeln V und VI 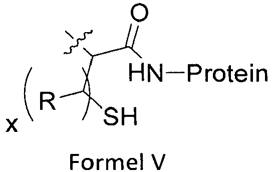 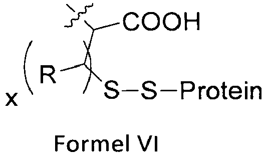 wobei jeweils unabhängig voneinander
R Wasserstoff oder einen linearen oder verzweigten Alkylrest mit 1 bis 8 Kohlenstoffatomen und
x 1 bis 6
bedeuten,
mindestens ein Protein kovalent angebunden ist,
wobei der Protein-funktionalisierter Film bevorzugt eine Dicke von 5 nm bis 500 nm, und/oder
das Copolymere oder das Polymere über Disulfidbrücken vernetzt ist und/oder mit dem Substrat kovalent verbunden ist, und/oder
der Gehalt des mindestens einen Proteins, bezogen auf die Gesamtmasse des Films von 0,01 bis 50 Gew%, bevorzugt 1 bis 20 Gew.% beträgt.

## Claims

1. A method of producing a protein-functionalized film in which a film comprising
at least one copolymer or polymer having an unhydrolyzed and/or hydrolyzed thiolactone functionalization and at least on protein is produced or provided, and
the at least one protein is covalently bonded to the at least one copolymer or polymer via the unhydrolyzed or hydrolyzed thiolactone functionalization,
**characterized in that**
the unydrolyzed thiolactone functionalization is selected from the group of residues having the general formula I shown below and the hydrolyzed thiolactone functionalization is preferably selected from the group of residues having the general formula II shown below 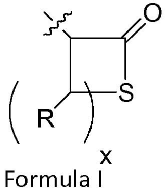 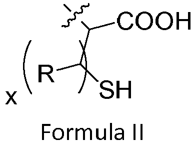 with the symbols meaning, respectively independently of one another
R hydrogen or a linear or branched alkyl residue having 1 to 8 carbon atoms; and
x 1 to 6.

2. A method in accordance with claim 1, **characterized in that** the film is produced directly on a substrate or by means of a Langmuir-Schaefer technique.

3. A method in accordance with the preceding claim, **characterized in that** the generation of the film takes place
on a substrate by coating with a doctor knife, spin coating and/or spray application from a solution of the at least one copolymer and/or polymer, preferably from an acidic or neutral solution, in particular from a solution having a pH of 5 to 7, and/or
by means of a Langmuir-Schaefer technique by spreading a solution of the at least one copolymer and/or polymer in a volatile solvent not miscible with water, in particular chloroform, over the surface of water or of an aqueous solution (aqueous subphase).

4. A method in accordance with the preceding claim, **characterized in that** the substrate is selected from the group comprising polymer films or polymer membranes, preferably polymer films or membranes surface-functionalized with amino groups, in particular films or membranes surface-functionalized with amino groups and made from polyacrylonitrile, polydimethylsiloxane, polymers on a cellulose base, polyvinyl alcohols, poly(hydroxyalkyl acrylates), in particular poly(2-hydroxyethyl acetate), poly(hydroxymethyl acrylate), and copolymers thereof, and inorganic substrates, in particular metal substrates, in particular coin metal substrates or substrates coated with coin metal, or inorganic substrates surface-functionalized with amino groups, in particular inorganic silicon wafers, glass substrates surface-functionalized with amino groups.

5. A method in accordance with one of the preceding claims, **characterized in that** the at least one protein
is applied to the surface of the film, in particular by spin coating and/or spray application of a solution of the at least one protein after the production or provision of the film of the at least one copolymer or polymer; and/or
is worked into the film of the copolymer and/or polymer during the production of the film by mixing the at least one protein with the at least one copolymer and/or polymer; and/or
in the case of the production of the film of the at least one copolymer and/or polymer by means of the Langmuir-Schaefer technique by adding the protein into the aqueous subphase before, during and/or after the production of the film of the at least one copolymer and/or polymer and the adsorption and/or absorption of the at least one protein on and/or in the film.

6. A method in accordance with one of the preceding claims, **characterized in that** the unydrolyzed thiolactone functionalization is preferably selected from the group of residues having the general formula I shown below and the hydrolyzed thiolactone functionalization is preferably selected from the group of residues having the general formula II shown below 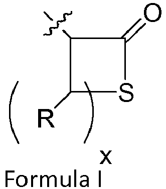 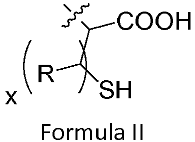 with the symbols meaning, respectively independently of one another
R hydrogen or a linear or branched alkyl residue having 1 to 8 carbon atoms; and
x 1 to 6;
and with R being hydrogen and x being 2 or 3.

7. A method in accordance with one of the preceding claims, **characterized in that** the copolymer contains repeat units or that the polymer is formed from repeat units having the following general formula III (unhydrolyzed) or IV (hydrolyzed) 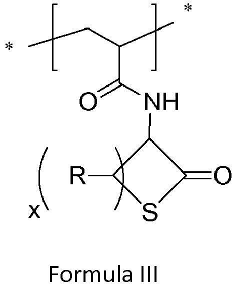 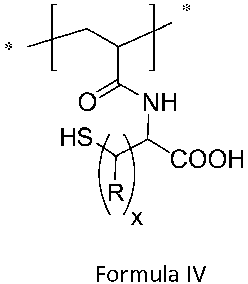 with the symbols meaning, respectively independently of one another
R hydrogen or a linear or branched alkyl residue having 1 to 8 carbon atoms; and
x 1 to 6;
and with preferably R being hydrogen and x being 2 or 3.

8. A method in accordance with one of the preceding claims, **characterized in that** the copolymer contains repeat units of the general formula shown below 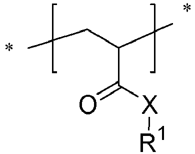 with the symbols meaning, respectively independently of one another
X NH, O or NR¹ and
R¹ a linear or branched alkyl residue having 1 to 8 carbon atoms or hydrogen.

9. A method in accordance with one of the preceding claims, **characterized in that** the copolymer is formed from the two following repeat units a) and b):
a) 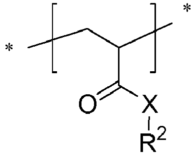 with the symbols meaning, respectively independently of one another
X NH;
R² one of the following residues 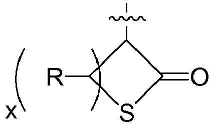 or 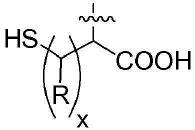
R hydrogen or a linear or branched alkyl residue having 1 to 8 carbon atoms; and
x 1 to 6;
and
b) 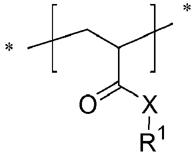 with the symbols meaning, respectively independently of one another
X NH, O or NR¹ and
R¹ a linear or branched alkyl residue having 1 to 8 carbon atoms or hydrogen,
with the repeat units a) and b) being present in statistically distributed form in the copolymer, and with in particular the molar portion of the repeat units a) amounting, with respect to the totality of the repeat units a) + b), to 5 to 50 mol%, preferably 10 to 40 mol%, and particularly preferably 15 to 30 mol%.

10. A method in accordance with one of the preceding claims, **characterized in that** the protein is an enzyme, in particular an enzyme selected from the group comprising aldolases, hydrolases, such as lipases, proteases, amidases, acylases, nitrilases, dehalogenases, isomerases, transferases, or a protein having a non-enzymatic function, in particular channel proteins and antibodies, and combinations thereof.

11. A method in accordance with one of the preceding claims, **characterized in that** the film is charged with 0.01 to 50 wt%, preferably 1 to 20 wt%, of the at least one protein.

12. A method in accordance with one of the preceding claims, **characterized in that** the film containing protein produced in the first step is stored over 0.1 to 24 h, preferably 0.1 to 8 h, in particular 0.1 to 2 h, for the covalent bonding of the protein to the at least one copolymer or polymer having an unhydrolyzed thiolactone functionalization.

13. A method in accordance with the preceding claim, **characterized in that** the storage takes place
at temperatures from 0 to 30°C, preferably 0 to 10°C; and/or
at a pH of 7.5 to 12, preferably 8 to 10; and/or
under the effect of an oxidation agent, for example hydrogen peroxide.

14. A method in accordance with any one of the claims 1 to 11, **characterized in that** the film containing a protein produced in the first step is treated under oxidizing conditions for the covalent bonding of the protein to the at least one copolymer or polymer having a hydrolyzed thiolactone functionalization, with disulfide bridges being produced between the protein's own thiol groups and the thiol groups of the hydrolyzed thiolactone.

15. A protein-functionalized film, containing at least one copolymer and/or polymer, to which at least one protein is covalently bonded via a spacer selected from the general formulas V and VI shown below 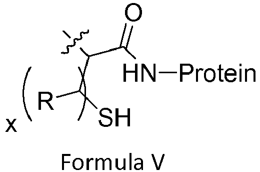 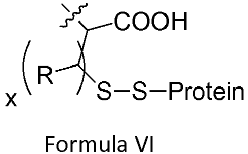 with the symbols meaning, respectively independently of one another
R hydrogen or a linear or branched alkyl residue having 1 to 8 carbon atoms; and
x 1 to 6;
with the protein-functionalized film preferably having a thickness of 5 nm to 500 nm; and/or
with the copolymer or the polymer being cross-linked via disulfide bridges and/or being covalently connected to the substrate; and/or
with the content of the at least one protein amounting, with respect to the total mass of the film, to from 0.01 to 50 wt%, preferably 1 to 20 wt%.

## Revendications

1. Procédé de fabrication d'un film fonctionnalisé par une protéine, dans lequel on produit ou on fournit un film contenant au moins un copolymère ou un polymère comportant une fonctionnalisation thiolactone non hydrolysée et/ou hydrolysée, ainsi qu'au moins une protéine, et
l'au moins une protéine est, par l'intermédiaire d'une fonctionnalisation thiolactone non hydrolysée ou hydrolysée, liée par liaison covalente à l'au moins un copolymère ou polymère,
**caractérisé en ce que**
la fonctionnalisation thiolactone non hydrolysée est choisie dans le groupe des radicaux ayant la formule générale I présentée ci-après, et la fonctionnalisation thiolactone hydrolysée est choisie dans le groupe des radicaux ayant la formule générale Il présentée ci-après 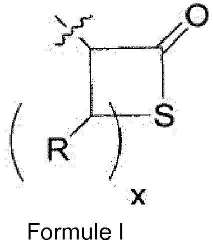 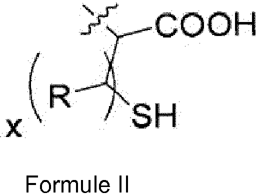 dans lesquelles, chacun indépendamment les uns des autres
R représente un atome d'hydrogène ou un radical alkyle à chaîne droite ou ramifiée ayant 1 à 8 atomes de carbone, et
x représente 1 à 6.

2. Procédé selon la revendication 1, **caractérisé en ce que** le film est produit directement sur un substrat ou par la technique de Langmuir-Schaefer.

3. Procédé selon la revendication précédente, **caractérisé en ce que** la production du film a lieu
sur un substrat par application à la racle, à la tournette et/ou par pulvérisation, dans une solution de l'au moins un copolymère et/ou polymère, de préférence dans une solution acide ou neutre, en particulier dans une solution ayant un pH de 5 à 7, et/ou
par la technique de Langmuir-Schaefer par étalement d'une solution de l'au moins un copolymère et/ou polymère sur un solvant volatil, non miscible à l'eau, de préférence le chloroforme, sur la surface d'eau ou d'une solution aqueuse (sous-phase aqueuse).

4. Procédé selon la revendication précédente, **caractérisé en ce que** le substrat est choisi dans le groupe consistant en les films ou membranes polymères, de préférence les films ou membranes polymères fonctionnalisés en surface par des groupes amino, en particulier les films ou les membranes fonctionnalisés en surface par des groupes amino, constitués de polyacrylonitrile, de polydiméthylsiloxane, les polymères à base de cellulose, de poly(alcools vinyliques), de poly(acrylates d'hydroxyalkyle), en particulier de poly(acrylate de 2-hydroxyéthyle), de poly(acrylate d'hydroxyméthyle), ainsi que les copolymères de ceux-ci, ainsi que les substrats inorganiques, en particulier les substrats métalliques, en particulier les substrats de métaux monétaires, ou les substrats revêtus d'un métal monétaire, ou les substrats inorganiques fonctionnalisés en surface par des groupes amino, en particulier les tranches de silicium inorganiques fonctionnalisées en surface par des groupes amino, les substrats de verre.

5. Procédé selon l'une des revendications précédentes, **caractérisé en ce que** l'au moins une protéine
après la production ou la fourniture du film de l'au moins un copolymère et/ou polymère, est appliquée sur la surface du film, en particulier par application à la tournette et/ou par pulvérisation d'une solution de l'au moins une protéine, et/ou
est incorporée dans le film du copolymère et/ou du polymère
pendant la production du film, par mélange de l'au moins une protéine avec l'au moins un copolymère et/ou polymère, et/ou
dans le cas de la production du film de l'au moins un copolymère et/ou polymère par la technique de Langmuir-Schaefer, par mise en place de la protéine dans la sous-phase aqueuse avant, pendant et/ou après production du film de l'au moins un copolymère et/ou polymère et adsorption et/ou absorption de l'au moins une protéine par et/ou dans le film.

6. Procédé selon l'une des revendications précédentes, **caractérisé en ce que** la fonctionnalisation thiolactone non hydrolysée est de préférence choisie dans le groupe des radicaux ayant la formule générale I présentée ci-après, et la fonctionnalisation thiolactone hydrolysée est de préférence choisie dans le groupe des radicaux ayant la formule générale II présentée ci-après 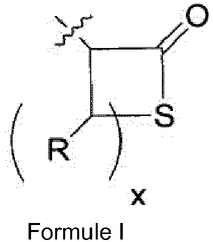 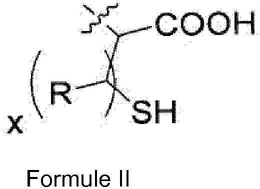 dans laquelle, chacun indépendamment les uns des autres,
R représente un atome d'hydrogène ou un radical alkyle à chaîne droite ou ramifiée ayant 1 à 8 atomes de carbone, et
x représente 1 à 6,
R représentant un atome d'hydrogène et x représentant 2 ou 3.

7. Procédé selon l'une des revendications précédentes, **caractérisé en ce que** le copolymère contient des motifs répétitifs, ou le polymère est formé de motifs répétitifs ayant la formule générale III (non hydrolysée) ou IV (hydrolysée) ci-après 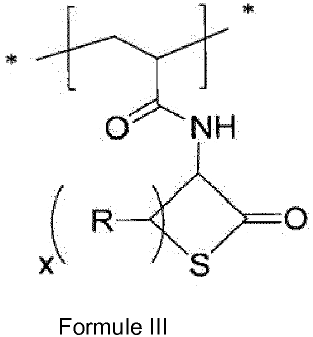 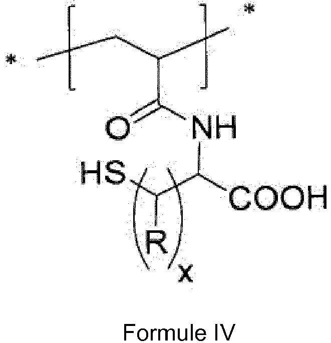 dans lesquelles, chacun indépendamment les uns des autres,
R représente un atome d'hydrogène ou un radical alkyle à chaîne droite ou ramifiée ayant 1 à 8 atomes de carbone, et
x représente 1 à 6,
et de préférence R représente un atome d'hydrogène et x représente 2 ou 3.

8. Procédé selon l'une des revendications précédentes, **caractérisé en ce que** le copolymère contient des motifs répétitifs ayant la formule générale présentée ci-après 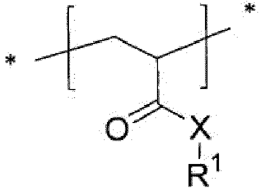 dans laquelle, chacun indépendamment les uns des autres,
X représente NH, O ou NR¹, et
R¹ représente un radical alkyle à chaîne droite ou ramifiée ayant 1 à 8 atomes de carbone ou un atome d'hydrogène.

9. Procédé selon l'une des revendications précédentes, **caractérisé en ce que** le copolymère est formé à partir des deux motifs répétitifs a) et b) ci-après :
a) 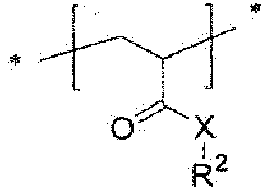 dans lequel, chacun indépendamment des autres,
X
représente NH,
R2 représente l'un des radicaux suivants : 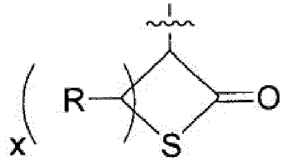 ou 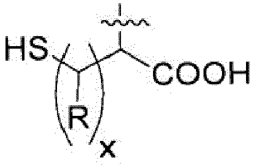
R représente un atome d'hydrogène ou un radical alkyle à chaîne droite ou ramifiée ayant 1 à 8 atomes de carbone, et
x représente 1 à 6, et
b) 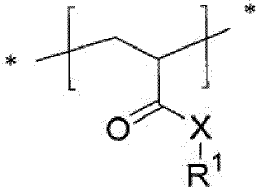 dans lequel, chacun indépendamment des autres,
X représente NH, O ou NR¹, et
R¹ représente un radical alkyle à chaîne droite ou ramifiée ayant 1 à 8 atomes de carbone ou un atome d'hydrogène,
dans lequel les motifs répétitifs a) et b) se présentent dans le copolymère statistiquement répartis, en particulier la proportion molaire des motifs répétitifs a), rapportée à la totalité des motifs répétitifs a) + b), étant de 5 à 50 % en moles, de préférence de 10 à 40 % en moles, d'une manière particulièrement préférée de 15 à 30 % en moles.

10. Procédé selon l'une des revendications précédentes, **caractérisé en ce que** la protéine est une enzyme, en particulier une enzyme choisie dans le groupe consistant en les aldolases, les hydrolases telles que par exemple les lipases, les protéases, les amidases, les acylases, les nitrilases, les déhalogénases, les isomérases, les transférases, ou une protéine ayant une fonction non enzymatique, en particulier les protéines canalaires et les anticorps, ainsi que les combinaisons de ceux-ci.

11. Procédé selon l'une des revendications précédentes, **caractérisé en ce que** le film est chargé de 0,01 à 50 % en poids, de préférence de 1 à 20 % en poids de l'au moins une protéine.

12. Procédé selon l'une des revendications précédentes, **caractérisé en ce que**, pour la liaison covalente de la protéine à l'au moins un copolymère ou polymère ayant une fonctionnalisation thiolactone non hydrolysée, le film contenant la protéine, produit dans la première étape, est stocké pendant 0,1 à 24 h, de préférence pendant 0,1 à 8 h, en particulier pendant 0,1 à 2 h.

13. Procédé selon la revendication précédente, **caractérisé en ce que** le stockage a lieu
à des températures de 0 à 30 °C, de préférence de 0 à 10 °C, et/ou
à un pH de 7,5 à 12, de préférence de 8 à 12, et/ou
sous l'action d'un oxydant, par exemple le peroxyde d'hydrogène.

14. Procédé selon l'une des revendications 1 à 11, **caractérisé en ce que**, pour la liaison covalente de la protéine à l'au moins un copolymère ou polymère ayant une fonctionnalisation thiolactone hydrolysée, le film contenant la protéine, produit dans la première étape, est traité dans des conditions oxydantes, des ponts disulfure étant produits entre les groupes thiol appartenant à la protéine et les groupes thiol de la thiolactone hydrolysée.

15. Film fonctionnalisé par une protéine, contenant au moins un copolymère et/ou polymère, auquel au moins une protéine est liée par liaison covalente par l'intermédiaire d'un espaceur choisi parmi les formules générales V et VI présentées ci-après 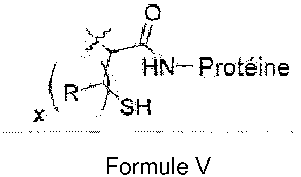 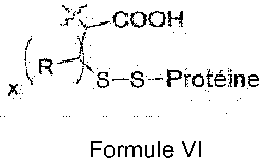 dans lesquelles, chacun indépendamment des autres,
R représente un atome d'hydrogène ou un radical alkyle à chaîne droite ou ramifiée ayant 1 à 8 atomes de carbone, et
x représente 1 à 6,
le film fonctionnalisé par une protéine présentant de préférence une épaisseur de 5 nm à 500 nm, et/ou
le copolymère ou le polymère étant réticulé par l'intermédiaire de ponts disulfure, et/ou étant lié par liaison covalente au substrat, et/ou
la teneur en l'au moins une protéine étant, par rapport à la masse totale du film, de 0,01 à 50 % en poids, de préférence de 1 à 20 % en poids.
